# EUROPEAN PATENT SPECIFICATION

(11) **EP 1 854 398 B1**
(45) Date of publication and mention of the grant of the patent: **05.08.2009**
(21) Application number: 07009161.6
(22) Date of filing: 07.05.2007
(51) Int. Cl.: A61B 1/005

(54) **Endoscope**
Endoskop
Endoscope

(30) Priority: 10.05.2006 JP 2006131499
(43) Date of publication of application: 14.11.2007
(73) Proprietor: Fujinon Corporation, Saitama-shi, Saitama (JP)
(72) Inventor: Torii, Yuichi, Saitama-shi Saitama (JP)
(74) Representative: Höhfeld, Jochen

(56) References cited:
- EP-A- 1 629 764
- EP-A2- 1 157 655
- DE-A1- 10 023 392
- JP-A- 11 019 031
- US-A- 4 809 680
- US-A- 4 832 003
- US-A- 5 976 074
- US-A1- 2001 023 313

## Description

### BACKGROUND OF THE INVENTION

### 1. Technical Field

The invention relates to an endoscope for use in medical care, improved in insertion-operability of an insertion part.

### 2. Related Art

An endoscope is provided by connecting a body operation portion that is gripped and operated by an operator's hand to a part inserted into a body cavity. The insertion part includes a flexible section that is bent in an arbitrary direction along an insertion direction from its portion connected to the body operation portion. A bending section and a distal-end hard section are sequentially connected to the flexible section. The distal-end hard section is formed with at least an illumination window and an observation window that form an endoscope observation mechanism. If necessary, the distal-end hard section is also formed with a treatment tool insertion channel that allows forceps and other treatment tools to be inserted therethrough, and also mounted with an injection nozzle for cleaning the observation window. The bending section is bendable by remote operation from the body operation portion in order to direct the distal-end hard section in a desired direction.

The insertion part of the endoscope is inserted into the body of a subject, and a constricted part exists in an insertion path of the insertion part. Accordingly, in order to relieve the subject's pain and to improve insertion operability of the insertion part, it is necessary to reduce the diameter of the insertion part and to shorten the length of the distal-end hard section.

Meanwhile, the internal wall of a body cavity constituting the insertion path of the insertion part in the body cavity is deformable to some extent. Therefore, if the insertion part is formed into a taper shape such that its distal end becomes thin, the insertion part can be inserted so as to push the constricted part wide. Thus, insertion operability is improved, and a burden to a subject is also relieved. The configurations in which the distal end of the insertion part is made thin in this way are disclosed in, for example, JP 2004-351138 A and JP Hei. 11-019031 A. In JP 2004-351138 A, a portion of the distal-end hard section on the side of an opening of a treatment tool insertion channel is formed into an obliquely cut shape. JP Hei.11-019031 A discloses a configuration in which the diameter of a'portion from the distal-end hard section to the bending section gets smaller continuously.

Here, both JP 2004-351138 A and JP Hei. 11-019031 A disclose a so-called optical endoscope in which an image guide made of an optical fiber bundle is used as an endoscope observation means which is mounted so as to face an observation window. An observed image in a body cavity is transmitted by the image guide. In this optical endoscope, an objective optical system and the image guide are used as observation means which are mounted to the distal-end hard section. As a result, the insertion part has an almost uniform outer diameter in its axial direction. Accordingly, there is no particular hindrance in forming the distal-end hard section into a desired shape. On the other hand, in the case of an electronic endoscope using an imaging means, it is necessary to arrange an imagingmeans including a solid-state imaging element and its circuit board in an imaging position of the objective optical system. For this reason, the section area occupied by the imaging means becomes larger than the section area occupied by the objective optical system. Moreover, the insertion part has not a uniform outer diameter in its axial direction but has a complicated shape. Furthermore, it is difficult to give a degree of freedom to an arrangement because each part constituting the imaging means is made of a hard member. Therefore, the structure of the optical endoscope cannot be simply applied thereto.

Also, if the portion of the distal-end hard section on the side of the opening of the treatment tool insertion channel is cut obliquely as disclosed in JP 2004-351138 A, the distal opening of the treatment tool insertion channel will be located in a position behind the observation window. As a result, when a treatment tool is intended to project from the treatment tool insertion channel, the treatment tool will not enter an observation field of view until it projects up to a certain length, which may result in a blind state. Accordingly, there is a problem in that the safety in the operation of the treatment tool is not secured. Moreover, if not only the distal-end hard section but also the bending section are formed into a taper shape as in JP Hei.11-019031 A, this shape affects the bending angle in the bending operation of the bending section. As a result, a sufficient bending angle will not be obtained. Accordingly, it is not desirable to form even the bending section into a taper shape.

Here, in an electronic endoscope in which a solid-state imaging means is provided in a distal-end hard section of an insertion part, JP Hei. 4-358114 A discloses such a configuration that a distal end of the distal-end hard section is formed into a taper shape. In JP Hei.4-358114 A, a portion of the distal-end hard section is made thin by partially cutting off the distal-end hard section.

In JP Hei.4-358114 A among the above-mentioned related arts, an unnecessary portion of the distal-end hard section is cutoff. This portion is cut off by only a length extremely limited in the axial direction. As a result, from the viewpoint that the distal-end hard section should be made thin towards the distal end as a whole, this configuration is still unsatisfactory. That is, in JP Hei.4-358114 A, although the imaging means including a solid-state imaging element is provided on the side of the proximal end of the objective optical system, the portion of the distal-end hard section to be cut off does not reach a portion where the imaging means is disposed. That is, the objective optical system is mounted, and the imaging means is mounted behind the objective optical system. Thus, the section area occupied in the distal-end hard section by the imaging means becomes larger than the section area occupied by the objective optical system. As a result, built-in objects are not provided in a portion to which the objective optical system is mounted. As such, a slight portion where built-in objects do not exist is cut off. For this reason, in JP Hei.4-358114 A, a taper portion is formed by the cut-off portion only at an extremely limited portion that is smaller than the axial length of the objective optical system. As a result, a satisfactory shape that is required to improve the operability of insertion of the insertion part into a constricted part, etc. cannot be obtained.

From US 4,832,003 an alternative approach to reduce the diameter of the insertion part is known, where the imaging means are mounted off-center inside the insertion part.

### SUMMARY OF THE INVENTION

The invention has been made in view of the above. The invention configures an endoscope, which is mounted with an imaging means including a solid-state imaging element and a circuit board as an observation means, so that almost the whole distal-end hard section of the insertion part is formed into a taper shape, to thereby facilitate the insertion part to pass through a constricted part in an insertion path, improve the insertion operability of the insertion part, and relieve subject's pains.

In order to achieve the above object, an endoscope includes a body operation portion, and an insertion part connected to the body operation portion. The insertion part includes a flexible section, a bending section and a distal-end hard section in this order from a side where the insertion part is connected to the body operation portion. The distal-end hard section includes a distal-end main body made of a hard member, and a connecting ring. The connecting ring includes a distal end portion fitted to the distal-end main body and a proximal end portion connected to the bending section. A distal end surface of the distal-end main body is formed with at least (i) an illumination window which an emitting end of a light guide faces and (ii) an observation window mounted to an objective optical system. An imaging means is arranged in an imaging position of the obj ective optical system. The imaging means is mounted inside the connecting ring, characterised in that the distal end portion of the connecting ring has the smallest wall thickness of the connecting ring, on a side where the connecting ring is connected to the distal-end main body . The wall thickness of the connecting ring gets larger from the distal end toward the proximal end portion of the connecting ring, on the side where the connecting ring is connected to the bending section. An outer peripheral surface of the connecting ring gets smaller in diameter continuously toward the distal end. The distal-end main body has a taper shape while being continuously connected to the outer peripheral surface of the connecting ring.

The distal-end hard section of the insertion part has at least an illumination means and an observation means fixedly mounted thereto. If necessary, the distal-end hard section is formed with a treatment tool guide part, and is provided with an injection nozzle for cleaning an observation window. For this reason, the distal-end hard section is configured to have a distal-end main body as a structural member. The distal-end hard section is connected to the bending section. As a structure for this connection between the distal-end hard section and the bending section, the distal-end main body may directly be connected to a portion of an angle ring in the bending section which is located at the most distal end. In the invention, the distal-end hard section and the bending section are connected to each other using a connecting ring. Also, the imaging means including a solid-state imaging element and a circuit board is located inside the connecting ring. Various members including the imaging means is mounted inside the connecting ring. In this case, it is natural that these members are arranged reasonably so that a dead space may not be produced. Thereby, the wall thickness of the connecting ring changes continuously in the axial direction. That is, the most distal end of the connecting ring fitted to the distal-end hard section will have a minimum thickness as a portion of the distal-end hard section on condition that required strength is given thereto. Also, a proximal portion, i.e., a portion of the bending section connected to the angle ring has the largest thickness. This thickness change appears in the outer peripheral surface, and the distal-end main body having a block structure is also reduced in diameter towards the distal end so as to be connected thereto. Thereby, the whole distal-end hard section gets thinner towards the distal end, that is, is formed into a taper shape. However, the bending section has a uniform outer diameter over its entire length.. This does not restrict the bending angle of the bending section.

The distal end surface of the distal-end hard section has the smallest diameter. In order to make the area of the distal end surface smaller, the positional relationship between the illumination window and observation window that constitute an endoscope observation means is taken into consideration. In order to suppress illumination unevenness to the minimum, two illumination windows are provided on both the right and left sides of the observation window. Both the illumination windows are arranged in positions as closer to the observation window as possible. This configuration makes it possible to reduce the area of the distal end surface in the distal-end hard section. As a result, the distal end can be made thin. The observation window is mounted with an objective optical system, and an imaging means is connected to the objective optical system. Also, the illumination window is mounted with an illuminating lens which diffuses illumination light, and light guide is arranged so as to face each illuminating lens. Since the imaging means arranged at the back of the objective optical system has a larger size than the objective optical system, the light guides may interfere with the imaging means when the light guides are pulled out straight rearward. Thus, the light guides are caused to extend in directions away from each other from their portions connected to the illumination windows towards their proximal ends, to thereby widen a space therebetween. Also, the imaging means includes a solid-state imaging element arranged between both the light guides, and a circuit board. The circuit board is wider than the solid-state imaging element. As a result, the light guides and the imaging means do not interfere with each other. The insertion part can be arranged without being particularly made large.

By forming almost the whole distal-end hard section of the insertion part into a taper shape, the insertion part can be inserted so as to push and expand a constricted part in the insertion path of the insertion part. As a result, the insertion operability of the insertion part is improved, and subject's pains can be relieved.

### BRIEF DESCRIPTION OF THE DRAWINGS

Fig. 1 shows the entire configuration of an endoscope according to an embodiment of the invention.
Fig. 2 is a sectional view of a distal end in an insertion part of the endoscope shown in Fig. 1.
Fig. 3 is a left side view of Fig. 2.
Fig. 4 is a sectional view taken along the line X-X of Fig. 2.
Fig. 5 is a sectional view taken along the line Y-Y of Fig. 2.
Fig. 6 is a sectional view taken along the line Z-Z of Fig. 2.

### DETAILED DESCRIPTION OF EMBODIMENTS OF THE INVENTION

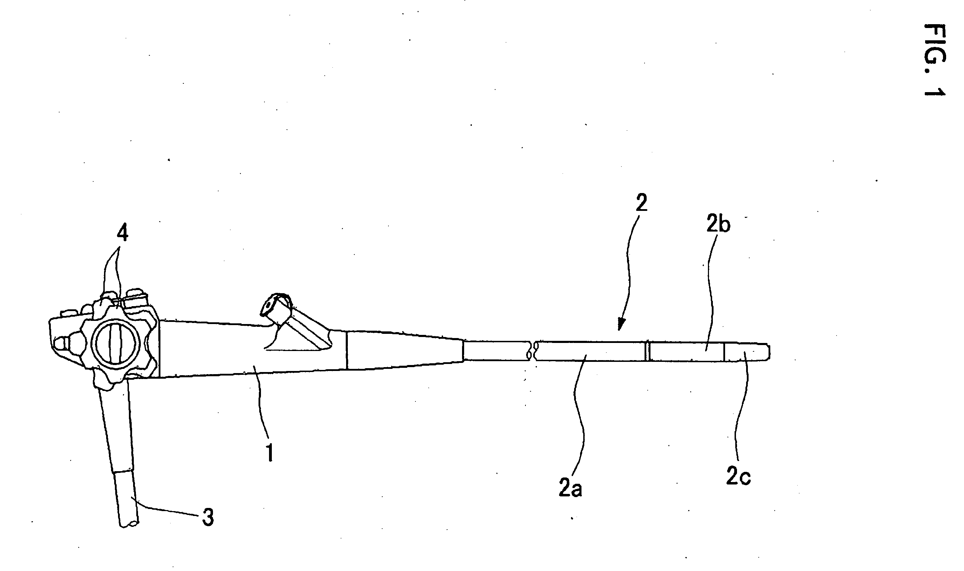

Hereinafter, an embodiment of the invention will be described with reference to the accompanying drawings. First, the entire configuration of an endoscope is shown in Fig. 1. In Fig. 1, reference numeral 1 denotes a body operation portion, reference numeral 2 denotes an insertion part inserted into a body cavity, and reference numeral 3 denotes a universal cord. These parts generally constitute an endoscope. Most of a length of the insertion part 2 from a side where the insertion part 2 is connected to the body operation portion 1 is occupied by a flexible section 2a. A bending section 2b and a distal-end hard section 2c are connected to this flexible section 2a. The bending section 2b is configured to be bendable by remote operation so that the distal-end hard section 2c may be directed in a desired direction. The bending operation is performed by an angle operation means 4 provided in the body operation portion 1. In addition, in this endoscope, the bending section 2b is configured so as to bend in two directions, i.e., an up direction and a down direction. However, the bending section 2b may also be configured so as to bend in four directions, i.e., up and down directions, and to right and left directions.

The insertion part 2 has a substantially uniform outer diameter from the flexible section 2a to a distal end of the bending section 2b, while the distal-end hard section 2c is formed into a taper shape that gets thinner as approaching the distal side. That is, among the insertion part 2, the bending section 2b has the largest outer diameter, and the outer diameter of the distal-end hard section 2c is reduced continuously from a connecting portion where the distal-end hard section 2c is connected to the bending section 2b towards the distal end. The distal-end hard section 2c is formed into a substantially truncated conical shape and a taper surface shape.

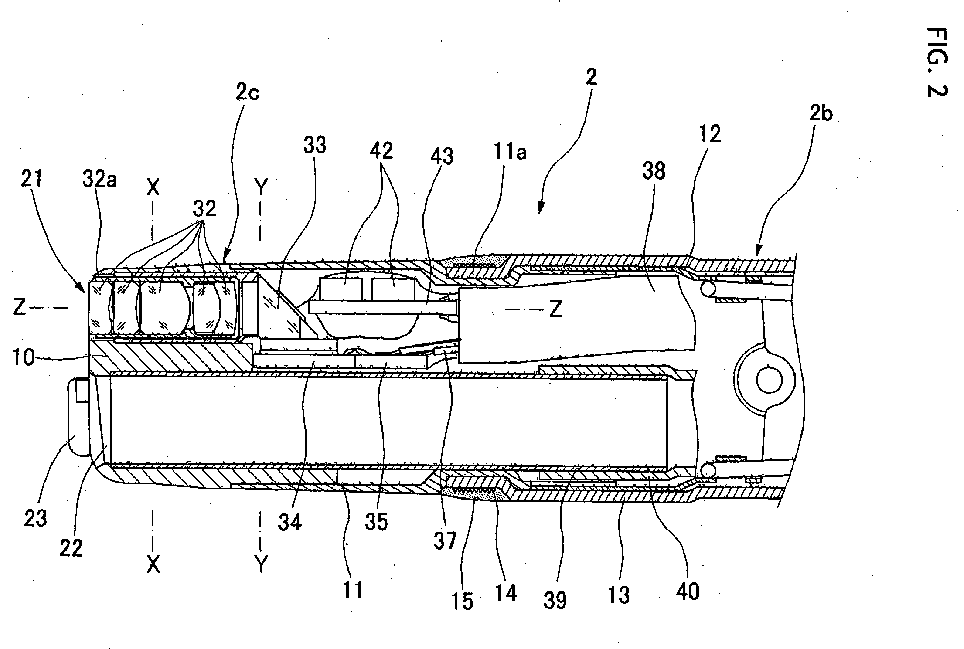

The cross section of the distal-end hard section 2c is shown in Fig. 2. In Fig. 2, reference numeral 10 denotes an distal-end main body, and reference numeral 11 denotes a connecting ring. The connecting ring 11 is fitted to the distal-end main body 10 at its distal portion, and is secured thereto with adhesive. Also, a proximal portion of the connecting ring 11 is fitted to a distal angle ring 12 that forms the bending section 2b. The connecting ring 11 and the angle ring 12 are fixed together by means of soldering. An annular recess 11a is formed in the vicinity of a connecting portion where the connecting ring 11 is connected to the angle ring 12. A distal end of an outer shell 13 of the bending section 2b extends to the recess 11a, and is secured thereto so that thread 14 is wound therearound and adhesive 15 is then piled. In addition, although not shown, a net is interposed between the angle ring 12 and the outer shell 13. The above structure is the outer structure of a portion from the distal-end hard section 2c to the bending section 2b. Various members are built in this structure. Each of the built-in members is fixedly supported by the distal-end main body 10 constituting the distal-end hard section 2c.

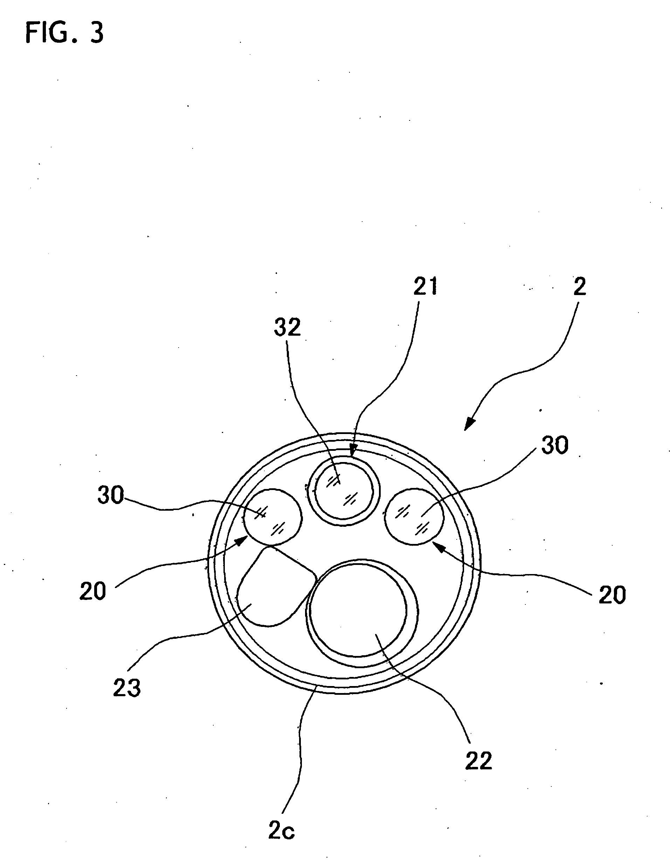

As shown in Fig. 3, a distal end surface of the distal-end main body 10 is formed with two illumination windows 20 and 20 and an observation window 21. The illumination windows 20 and 20 are arranged on both sides of the observation window 21 with sandwiching the observation window 21. Also, a treatment tool guide part 22 is opened in a position substantially below the observation window 21. An injection nozzle 23 is provided in a position that is in an obliquely downward direction with respect to the observation window 21. The injection nozzle 23 has an injection port that faces the observation window 21.

Accordingly, a body cavity can now be observed through the observation window 21 under the illumination light emitted from the illumination windows 20 and 20. Also, when affected parts are discovered by the observation through the observation window 21, treatment tools, such as forceps and a high-frequency treatment tool, will be guided from the treatment tool guide part 22. Appropriate treatment will then be performed. A good observation field of view should always be obtained through the observation window 21. If the observation window 21 is soiled with body fluid, etc. , washing water is injected from the injection nozzle 23 to flush soils, and then pressurized air is injected to remove water drops adhering to the observation window 21.

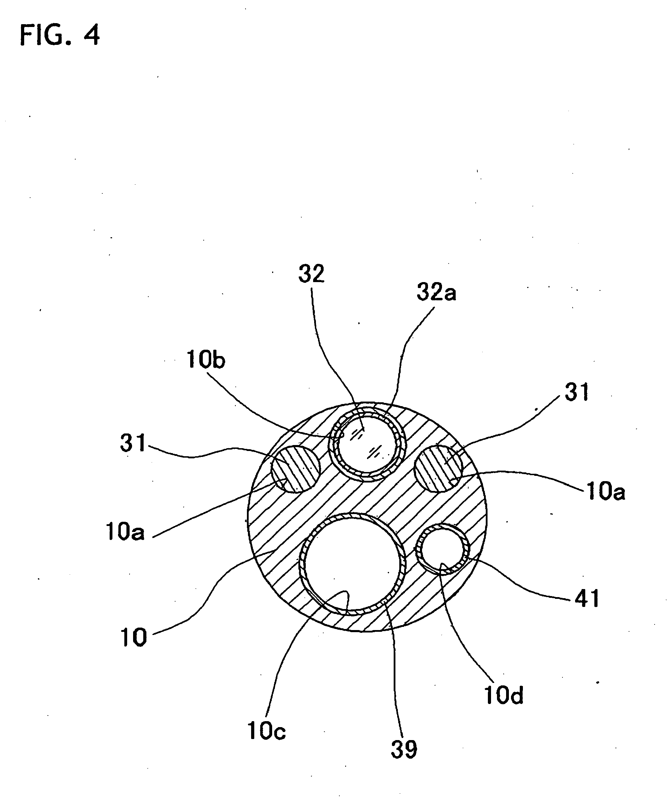

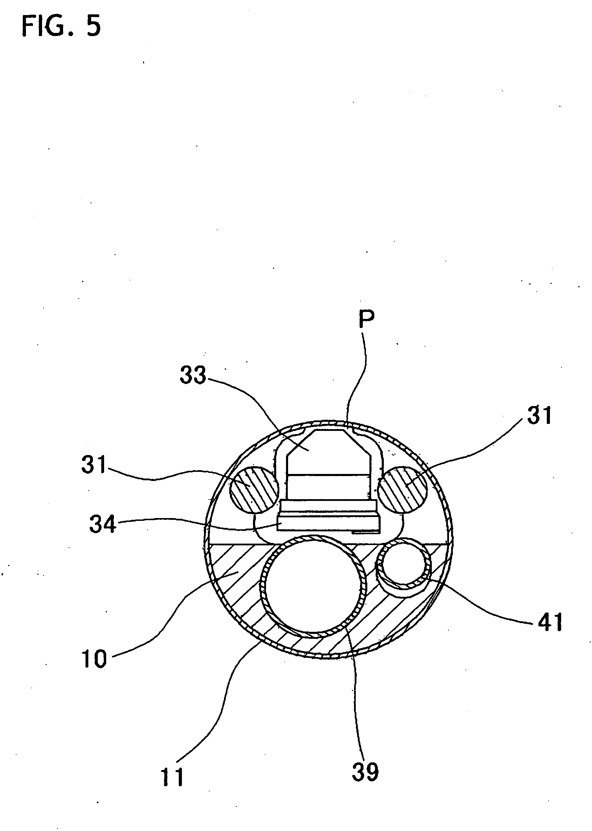

As clear from Figs. 2 and 3 and as also shown in Figs. 4 and 5, each illumination window 20 is mounted with an illuminating lens 30, and an emitting end of a light guide 31 faces the illuminating lens 30. Thereby, the illumination light emitted from the light guide 31 is diffused by the illuminating lens 30. The observation window 21 is mounted with a lens barrel 32a mounted with an objective optical system 32. A prism 33 secured to an end of the lens barrel 32a is connected to a rear end of the objective optical system 32. Thereby, the optical path of the objective optical system 32 is bent by 90 degrees. Also, a solid-state imaging element 34 is provided in an imaging position of the objective optical system 32. The solid-state imaging element 34 is connected to a circuit board 35. Accordingly, these solid-state imaging elements 34 and the circuit board 35 constitute an imaging means. The circuit board 35 is provided with a number of electrodes 36. Wiring lines 37 are connected to the electrodes 36, respectively. The wiring lines 37 are bundled into one cable 38. The cable 38 extends into the universal cord 3 via the body operation portion 1 from the insertion part 2 along with the light guide 31. Also, a connecting pipe 39 is inserted into the treatment tool guide part 22. A flexible treatment tool insertion tube 40 is connected to the connection pipe 39. A treatment tool insertion path is formed by the treatment tool guide part 22, the connecting pipe 39, and the treatment tool insertion tube 40. Meanwhile, a fluid supply tube 41 is connected to the injection nozzle 23.

The above-mentioned built-in objects are provided within the distal-end hard section 2c. These objects are supported by the distal-end main body 10. For this purpose, as clear from Fig. 4, a predetermined number of through-holes are provided in the distal-end main body 10. The built-in objects are inserted into the corresponding through holes. That is, through-holes 10a and 10a are inserted parts for the light guides 31. The lens barrel 32a of the objective optical system 32 is inserted through a through-hole 10b. Also, a through hole 10c is an inserted part for the connecting pipe 39 connected to the treatment tool guide part 22. A through-hole 10d is an inserted part for the fluid supply tube 41. In order to shorten the length of the distal-end hard section 2c, a minimum length required to stably support each of these built-in objects is provided as an axial length of the distal-end main body 10.

Of the above built-in objects, the light guides 31 and the fluid supply tube 41 do not change in section area at mounting portions where the light guides 31 and the fluid supply tube 41 are mounted to the distal-end main body 10 and at portions closer to the proximal end than the mounted portions. In the treatment tool insertion path, the treatment tool insertion tube 40 is connected to the connecting pipe 39. Accordingly, the diameter of the treatment toll insertion tube 40 becomes slightly large at the connecting portion where the connecting pipe 39 is connected to the treatment tool insertion tube 40. Also, although not shown, the fluid supply tube 41 connected to the injection nozzle 23 is also connected to a flexible tube in the vicinity of a connecting portion where the connecting pipe 39 and the treatment tool insertion tube 40 are connected to each other. The structure of the observation means changes remarkably at a portion supported by the distal-end main body 10 and at a portion closer to the proximal end than the supported portion. The proximal portion requires a significantly broad space. The distal-end hard section 2c includes the distal-end main body 10 and the connecting ring 11 in order to secure the space for mainly allowing the observation means to be mounted. That is, since the lens barrel 32a is provided at the distal-end main body 10, the through-hole 10b through which the lens barrel 32a passes is formed. However, on the side of the proximal end of the lens barrel 32a, the prism 33 is provided, and the solid-state imaging element 34 and its circuit board 35 are also provided. Moreover, the configuration shown in Fig. 2 is also provided with a sub substrate 43 on which electronic components 42 are mounted. These are arranged inside the connecting ring 11, and are mounted further ahead than an arrangement portion of the annular recess 11a to which the distal end of the outer shell 13 of the bending section 2b is fixed, to thereby secure an installation space for the observation means inside the connecting ring 11. On the other hand, the connecting portion between the connecting pipe 39 and the treatment tool insertion tube 40 that constitute the treatment tool insertion path is arranged in a proximal portion of the connecting ring 11 beyond the recess 11a.

As already described, in the insertion part 2, the distal-end main body 10 constituting the distal-end hard section 2c of the insertion part 2 is formed into such a taper shape that the distal-end hard section 2c becomes the smallest in diameter at its most distal end surface, and that the distal-end hard section 2c is continuously increased in diameter toward the proximal end, that is, toward the side where the distal-end main body 10 is connected to the connecting ring 11. Also, the connecting ring 11 is configured such that its thickness is the smallest at a fitting portion where the connecting ring 11 is fitted to the distal-end main body 10 and continuously increases toward the proximal end, that is, toward the side where the connecting ring 11 is connected to the bending section 2b. Moreover, the inner diameter of the connecting ring 11 does not change substantially to a portion where the recess 11a is formed, but only the outer diameter thereof gets large. Accordingly, the outer surface of the connecting ring 11 is formed into a taper surface shape. Also, the taper surface of the connecting ring 11 is formed into a taper shape that substantially continues to the tapered surface of the distal-end main body 10. No step is formed therebetween. The taper surface of the connecting ring 11 and the tapered surface of the distal-end main body 10 are formed into a series of taper surface shape to a portion where the recess 11a is formed.

By configuring the endoscope in this way, even if there is a constricted part on the way of the insertion path when the insertion part 2 is inserted into a body cavity, it is easy to cause the most distal end to pass through the constricted part so as to push and expand the constricted part wide so long as the most distal end of the distal-end hard section 2c is caused to enter the constricted part. At this time, a pain to be given to a subject will be suppressed to the minimum. Moreover, insertion operability is further improved by reducing the diameter of the distal-end hard section 2c and shortening the length of the hard section. Also, the smaller the diameter of the most distal end of the distal-end hard section 2c, the better the insertion performance to the constricted part becomes. For this purpose, the illumination windows 20 and 20 are made closer to the observation window 21, to thereby reduce the area of the distal end surface in the distal-end hard section 2c.

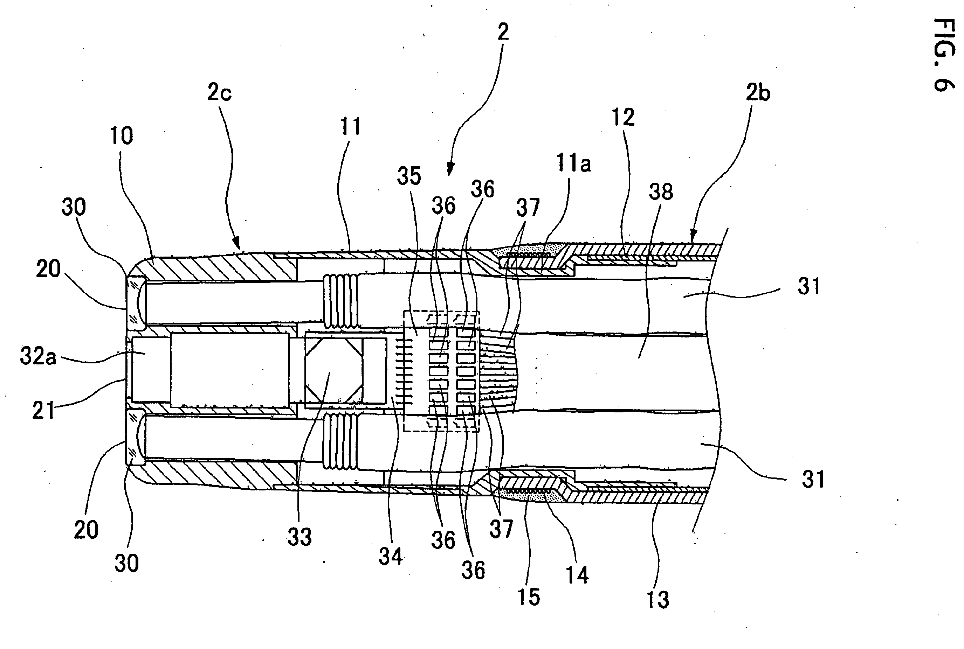

Meanwhile, the prism 33 is coupled to the lens barrel 32a of the objective optical system 32 mounted to the observation window 21. The solid-state imaging element 34 is connected to the bottom surface of the prism 33. The solid-state imaging element 34 is connected to the circuit board 35. When the illumination window 20 is brought close to the observation window 21, there is a possibility that the light guide 31 extending from the illumination window 20 may interfere with the prism 33, the solid-state imaging element 34 or the circuit board 35. In order to avoid such a situation, both the light guides 31 and 31, which face both the illumination windows 20 and 20 arranged on both sides of the observation window 21, extend in directions away from each other toward the proximal end as shown in Fig. 6. Also, the circuit board 35 is wider in a direction perpendicular to the axial direction of the insertion portion 2 than the solid-state imaging element 34. This structure prevents the light guide 31 from interfering with each of the parts constituting the observation means, and prevents optical fibers from being broken because of the light guide 31 being pressed. Also, since an arrangement portion for the electrodes 36 is made wide, the wiring lines 37 can be connected to the circuit board 35 reasonably.

The recess 11a is arranged in a portion of the connecting ring 11 closer to the proximal end than the circuit board 35. The inner diameter of the connecting ring 11 is small. Thus, both the light guides 31 and 31 are bent in directions in which they approach each other again from a portion where the cable 38 extends and beyond the circuit board 35. This structure makes it possible for the light guides 31 and 31 to smoothly pass through the position of the recess 11a, which is provided to fix the outer shell 13.

The connecting ring 11 has an inner diameter required to secure a sufficient space to accommodate various built-in objects. Sufficient strength can be given to the connecting ring 11 because the wall thickness of the connecting ring 11 on the side of the proximal end is large. However, since the wall thickness of the connecting ring 11 on the side of the distal end is small, the connecting ring 11 is weakened. Then, even the most thin-walled portion of the distal end of the connecting ring 11 is configured to have such strength that deformation is not caused by an ordinarily acting external force. Here, since the distal end of the connecting ring 11 is fitted to the distal-end main body 10, the distal end can be made considerably thin-walled so long as the connecting ring 11 has the minimum strength in the fitting state. However, the connecting ring 11 is not necessarily fitted to the distal-end main body 10 over its entire periphery. For example, as shown in Fig. 5, the distal-end main body 10 is cut out at a portion P where the top of the prism 33 constituting the observation means is arranged. With this configuration, a mounting portion for the observation means can be small-sized. Thus, strength reduction is not particularly caused in the fitting portion of the connecting ring 11 by making the cutout portion P extremely narrow.

## Claims

1. An endoscope comprising:
a body operation portion (1); and
an insertion part (2) connected to the body operation portion, wherein:
the insertion part comprises a flexible section (2a), a bending section (2b) and a distal-end hard section (2c) in this order from a side where the insertion part is connected to the body operation portion,
the distal-end hard section comprises
a distal-end main body (10) made of a hard member and having a taper shape and
a connecting ring (11) including a distal end portion fitted to the distal-end main body and a proximal end portion connected to the bending section,
wherein a distal end surface of the distal-end main body is formed with at least (i) an illumination window (20) which is faced by an emitting end of a light guide (31) and (ii) an observation window (21) mounted to an objective optical system (32),
and an imaging means (34, 35) is arranged in an imaging position of the objective optical system,
the imaging means being mounted inside the connecting ring, **characterised in that** the distal end portion of the connecting ring has the smallest wall thickness of the connecting ring, on a side where the connecting ring is connected to the distal-end main body,
the wall thickness of the connecting ring gets larger from the distal end portion toward a proximal end of the connecting ring, on the side where the connecting ring is connected to the bending section,
an outer peripheral surface of the connecting ring gets smaller in diameter continuously toward the distal end portion thus forming a taper surface shape, and wherein further the taper surface of the connecting ring (11) is formed into a taper shape that continues to the tapered surface of the distal-end main body (10)

2. The endoscope according to claim 1, wherein:
the two illumination windows (20) are arranged on both sides of the observation window (21) so as to sandwich the observation window,
the light guides (31) extend in directions away from each other from a side where the light guides are connected to the illumination windows towards a proximal end side,
the imaging means (34, 35) comprises
a solid-state imaging element (34) arranged between both the light guides, and
a circuit board (35), and
the circuit board is wider in a direction perpendicular to an axial direction of the insertion portion than the solid-state imaging element.

## Patentansprüche

1. Endoskop umfassend:
einen Körperbehandlungs-Teilabschnitt (1); und
einen Einführungsteil (2), welcher mit dem Körperbehandlungs-Teilabschnitt verbunden ist, wobei:
der Einführungsteil einen flexiblen Abschnitt (2a), einen Biegungsabschnitt (2b) und einen harten, Distalende-Abschnitt (2c) in dieser Reihenfolge, ausgehend von der Seite an der der Einführungsteil mit dem Körperbehandlungs-Teilabschnitt verbunden ist, umfasst,
der harte, Distalende-Abschnitt umfasst
einen Distalende-Hauptkörper (10), welcher aus einem harten Element gemacht ist und eine abgeschrägte Form besitzt, und
einen Verbindungsring (11) umfassend einen Distalende-Teilabschnitt, welcher mit dem Distalende-Hauptkörper und einem proximalen, mit dem Biegungsabschnitt verbundenen End-Teilabschnitt angepasst ist,
wobei eine distale Endoberfläche des Distalende-Hauptkörpers mit zumindest (i) einem Beleuchtungsfenster (20), welchem ein emittierendes Ende eines Lichtleiters (31) gegenüber liegt, und (ii) einem Beobachtungsfenster (21), welches an einem optisches Objektivsystem (32) befestigt ist, ausgebildet ist, und
ein Bildgebungsmittel (34, 35) an der Bildgebungs-Position des optischen Objektivsystems angeordnet ist,
das Bildgebungsmittel innerhalb des Verbindungsrings befestigt ist,
**dadurch gekennzeichnet, dass**
der distale End-Teilabschnitt des Verbindungsrings die kleinste Wanddicke des Verbindungsrings an einer Seite aufweist, an der der Verbindungsring mit dem Distalende-Hauptkörper verbunden ist,
die Wanddicke des Verbindungsrings von dem distalen End-Teilabschnitt zu einem proximalen Ende des Verbindungsrings auf der Seite, an der der Verbindungsring mit dem Biegungsabschnitt verbunden ist, zunimmt,
der Durchmesser einer äußeren Umfangsoberfläche des Verbindungsrings in Richtung des distalen End-Teilabschnitts kontinuierlich kleiner wird, wodurch eine abgeschrägte Oberflächenform ausgebildet wird, und
wobei weiterhin die abgeschrägte Oberfläche des Verbindungsrings (11) mit einer abgeschrägten Form ausgebildet ist, die sich zu der abgeschrägten Oberfläche des Distalende-Hauptkörpers (10) fortsetzt.

2. Endoskop nach Anspruch 1, wobei:
die zwei Beleuchtungsfenster (20) derart auf beiden Seiten des Beobachtungsfensters (21) angeordnet sind, dass diese eine Sandwich-Struktur mit dem Beobachtungsfenster bilden,
die Lichtleiter (31) erstrecken sich in Richtungen voneinander weg von einer Seite an der die Lichtleiter mit den Beleuchtungsfenstern verbunden sind zu einer proximalen Endseite,
das Bildgebungsmittel (34, 35) umfasst
ein Festkörper-Bildgebungselement (34), welches zwischen den beiden Lichtleitern angeordnet ist, und
eine Platine (35) und
die Platine ist in einer Richtung senkrecht zu einer axialen Richtung des Einführungsteils breiter als das Festkörper-Bildgebungselement.

## Revendications

1. Endoscope comprenant :
une portion d'opération du corps (1) ; et
une partie d'insertion (2) raccordée à la portion d'opération du corps, dans lequel :
la partie d'insertion comprend une section flexible (2a), une section de fléchissement (2b) et une section dure d'extrémité distale (2c) dans cet ordre depuis un côté où la partie d'insertion est raccordée à la portion d'opération de corps,
la section dure d'extrémité distale comprend
un corps principal d'extrémité distale (10) constitué d'un organe dur et ayant une forme conique, et
une bague de raccord (11) incluant une portion d'extrémité distale ajustée au corps principal d'extrémité distale et une portion d'extrémité proximale raccordée à la section de fléchissement,
dans lequel une surface d'extrémité distale du corps principal d'extrémité distale est formée avec au moins (i) une fenêtre d'éclairage (20) qui est en regard d'une extrémité émettrice d'un guide de lumière (31) et (ii) une fenêtre d'observation (21) montée sur un système optique d'objectif (32),
et un moyen d'imagerie (34, 35) est agencé dans une position d'imagerie du système optique d'objectif,
le moyen d'imagerie étant monté à l'intérieur de la bague de raccord, **caractérisé en ce que** la portion d'extrémité distale de la bague de raccord a la plus petite épaisseur de paroi de la bague de raccord, sur un côté où la bague de raccord est raccordée au corps principal d'extrémité distale,
l'épaisseur de paroi de la bague de raccord devient plus grande de la portion d'extrémité distale vers une extrémité proximale de la bague de raccord, sur le côté où la bague de raccord est raccordée à la section de fléchissement,
une surface périphérique externe de la bague de raccord devient plus petite en diamètre de façon continue vers la portion d'extrémité distale formant une forme ainsi une surface conique et dans lequel en outre, la surface conique de la bague de raccord (11) est formée en une forme conique qui continue vers la surface conique du corps principal d'extrémité distale (10).

2. Endoscope selon la revendication 1, dans lequel :
les deux fenêtres d'éclairage (20) sont agencées sur les deux côtés de la fenêtre d'observation (21) de façon à prendre en sandwich la fenêtre d'observation,
les guides de lumière (31) s'étendent dans des directions en éloignement l'un de l'autre depuis le côté où les guides de lumière sont raccordés aux fenêtres d'éclairage vers un côté d'extrémité proximale,
le moyen d'imagerie (34, 35) comprend
un élément d'imagerie semi-conducteur (34) agencé entre les deux guides de lumière, et
une carte de circuit (35), et
la carte de circuit est plus large dans une direction perpendiculaire à une direction axiale de la portion d'insertion que l'élément d'imagerie semi-conducteur.
